(19) **Europäisches Patentamt**

**European Patent Office**

**Office européen des brevets**

(11) **EP 1 734 132 A1**

(12) **EUROPEAN PATENT APPLICATION**
published in accordance with Art. 158(3) EPC

(43) Date of publication:
**20.12.2006 Bulletin 2006/51**

(51) Int Cl.:
***C12Q 1/44*** *(2006.01)* *G01N 33/49* *(2006.01)*

(21) Application number: **05728780.7**

(86) International application number:
**PCT/JP2005/006669**

(22) Date of filing: **05.04.2005**

(87) International publication number:
**WO 2005/098026 (20.10.2005 Gazette 2005/42)**

(84) Designated Contracting States:
**AT BE BG CH CY CZ DE DK EE ES FI FR GB GR HU IE IS IT LI LT LU MC NL PL PT RO SE SI SK TR**

(30) Priority: **05.04.2004 JP 2004111051**

(71) Applicant: **YAMASA CORPORATION**
**Choshi-shi,**
**Chiba 288-0056 (JP)**

(72) Inventor: **HAMAOKI, Masaru**
**Chiba 288-0812 (JP)**

(74) Representative: **Hartz, Nikolai**
**Wächtershauser & Hartz**
**Weinstrasse 8**
**80333 München (DE)**

(54) **METHOD OF DETERMINING CARBONIC ANHYDRASE I ACTIVITY**

(57) To provide a method for specifically determining CA isozyme activity.

The method for determining hydrolase activity of carbonic anhydrase I (CAI) in a sample, which is **characterized in that** the method employs, as a substrate or a combination of a substrate and an inhibitor, any of the following (A) to (E):

(A) Substrate: a substrate having higher reactivity with CAI than with CAII;

(B) Substrate: a substrate having higher reactivity with CAI than with CAII,

Inhibitor: an inhibitor inhibiting a hydrolase other than CA, and

an optional drug for enhancing inhibitory activity of the inhibitor;

(C) Substrate: a substrate having higher reactivity with CAI than with CAII, and

Inhibitor: a CA inhibitor inhibiting both CAI and CAII;

(D) Substrate: a substrate having reactivity with both CAI and CAII, and

Inhibitor: a CA inhibitor inhibiting CAI more potently than CAII; and

(E) Substrate: a substrate having higher reactivity with CAI than with CAII, and

Inhibitor: a CA inhibitor inhibiting CAI more potently than CAII.

EP 1 734 132 A1

**Description**

Technical Field

The present invention relates to a method for determining carbonic anhydrase I (CAI; also called carbonic anhydrase B) activity and to a kit for determining the activity.

Background Art

Carbonic anhydrase (CA) I and CAII are present in erythrocytes. In clinical examinations, the total level of CAI and CAII serves as a basis of the diagnosis of conditions such as iron-deficrency anemia and respiratory distress syndrome. Differing from total CA level, application of the level of CAI, which is an isozyme of CAs, is suggested for clinical diagnosis of hyperthyroidism, hypothyroidism, etc. (Non-Patent Document 1).
One possible method for specifically determining CA isozyme activity is the immunodiffusion method. However, the method is not employed in clinical examinations, since the method includes cumbersome steps and is time consuming.
In addition to carbonic anhydrase activity, CA has hydrolase activity. When the hydrolase activity is measured, CA isozyme activity may be determined through a simple method such as spectrometry (Non-Patent Document 2). However, in the case of clinical specimens, which contain large amounts of hydrolases, other than CA, and under the conditions where a significant amount of an added substrate is decomposed by the above enzymes (e.g., an added substrate is rapidly decomposed by the enzymes), determination of specific CA isozyme hydrolase activity has been considered impossible.
Under such circumstances, there has been reported another approach for specifically determining CAI activity including determination of hydrolase activity before and after immunoadsorption by use of an antiserum against CAI (Non-Patent Document 3). However, the method is not simple in practice.
Meanwhile, total CA activity of all hydrolase species can be determined by use of acetazolamide, which is a CA-specific inhibitor (Non-Patent Document 4). However, hitherto, no method for specifically determining CA isozyme activity has been reported.

Non-Patent Document 1: Tohoku J. Exp. Med., 1996, 178, 345-356
Non-Patent Document 2: The Journal of Biological Chemistry, 1967; 242(18): 4221-4229
Non-Patent Document 3: Clinica Chimica Acta, 60(1975), 347-353
Non-Patent Document 4: The Journal of Biological Chemistry, 1966; 241(10): 5137-5149

**Disclosure of the Invention**

Problems to be Solved by the Invention

An object of the present invention is to provide a method for specifically determining CA isozyme activity. Means for Solving the Problems
The present inventor has carried out extensive studies, and has found that CA isozyme activity, particularly CAI hydrolase activity, can be specifically determined by employing a specific substrate, or a combination of a specific substrate and a specific inhibitor with an optional inhibition-enhancer. The inventor has also found that the technique can be employed for the analysis of clinical specimens, such as whole blood, containing large amounts of hydrolases other than CA. The present invention has been accomplished on the basis of these findings.
Accordingly, the present invention provides a method for determining hydrolase activity of carbonic anhydrase I (CAI) in a sample, characterized in that the method employs, as a substrate or a combination of a substrate and an inhibitor, any of the following (A) to (E):

(A) Substrate: a substrate having higher reactivity with CAI than with CAII;
(B) Substrate: a substrate having higher reactivity with CAI than with CAII,
Inhibitor: an inhibitor inhibiting a hydrolase other than CA, and
an optional drug for enhancing inhibitory activity of the inhibitor;
(C) Substrate: a substrate having higher reactivity with CAI than with CAII, and
Inhibitor: a CA inhibitor inhibiting both CAI and CAII;
(D) Substrate: a substrate having reactivity with both CAI and CAII, and
Inhibitor: a CA inhibitor inhibiting CAI more potently than CAII; and
(E) Substrate: a substrate having higher reactivity with CAI than with CAII, and
Inhibitor: a CA inhibitor inhibiting CAI more potently than CAII.

The present invention also provides a kit for determining hydrolase activity of CAI in a sample, characterized in that the kit comprises any of the aforementioned (A) to (E) as a substrate, a combination of a substrate and an inhibitor, or a combination of a substrate, an inhibitor, and an inhibition-enhancer.

Effects of the Invention

The determination method of the present invention employs a specific substrate, a combination of a specific substrate and a specific inhibitor, or a combination of a specific substrate, a specific inhibitor, and a specific inhibition-enhancer. Therefore, hydrolase activity of CAI in a sample can be determined specifically, selectively, or substantially. The method of the invention can be performed in a simple manner. In addition, the method can be applied to clinical specimens or other conditions where large amounts of hydrolases, other than CA, are present and a significant amount of an added substrate is decomposed by such enzymes (specifically, an added substrate is rapidly decomposed by the enzymes), and this makes the method a practical CAI activity determination method. Among the above combinations, a determination method employing (B) above; i.e., a combination of a substrate, an inhibitor, and an inhibition-enhancer, is remarkably useful, since the method can exclude the effects of hydrolases other than CA, and enables specific determination of CAI hydrolase activity through a single measurement operation.

Best Modes for Carrying Out the Invention

In the present invention, no particular limitation is imposed on the sample to be analyzed, and any sample may be analyzed so long as it contains an erythrocyte component. Preferably, the sample is an erythrocyte lysate. In addition to an erythrocyte component, the sample may contain other blood components.
As mentioned above, the present invention is directed to a method for determining hydrolase activity of CAI in a sample, characterized in that the method employs any of the following (A) to (E) as a substrate, a combination of a substrate and an inhibitor or a combination of a substrate, an inhibitor and an inhibition-enhancer:

(A) Substrate: a substrate having higher reactivity with CAI than with CAII;
(B) Substrate: a substrate having higher reactivity with CAI than with CAII,
Inhibitor: an inhibitor inhibiting a hydrolase other than CA, and
an optional drug for enhancing inhibitory activity of the inhibitor;
(C) Substrate: a substrate having higher reactivity with CAI than with CAII, and
Inhibitor: a CA inhibitor inhibiting both CAI and CAII;
(D) Substrate: a substrate having reactivity with both CAI and CAII, and
Inhibitor: a CA inhibitor inhibiting CAI more potently than CAII; and
(E) Substrate: a substrate having higher reactivity with CAI than with CAII, and
Inhibitor: a CA inhibitor inhibiting CAI more potently than CAII.

The method of the present invention includes the following two methods: a method comprising determining hydrolase activity (specifically, esterase activity) of a sample by use of the aforementioned (A) or (B) as a substrate, a combination of a substrate and an inhibitor, or a combination of a substrate, an inhibitor, and an enhancer, and employing the thus-determined hydrolase activity as CAI hydrolase activity, and a method comprising determining hydrolase activity (specifically, esterase activity) of a sample by use of any of the aforementioned (C) to (E) in the presence and in the absence of a CA inhibitor, and employing the difference between the two values as CAI hydrolase activity.
In the present invention, the "substrate having higher reactivity with CAI than with CAII" refers to a substrate which reacts with CAI in an amount, per amount of enzyme protein, twice or more the amount of substrate reacting with CAII, through controlling conditions such as substrate concentration and reaction time. Examples of the substrate include esters such as 2-hydroxy-5-nitro-α-toluenesulfonic acid sultone, o-nitrophenyl esters, p-nitrophenylthio esters, and β-naphthyl esters. Of these, o-nitrophenyl esters are particularly preferred. These esters are preferably derived from a fatty acid, more preferably a C1 to C6 fatty acid. Specifically, o-nitrophenyl acetate is preferred.
The "substrate having reactivity with both CAI and CAII" refers to a substrate which reacts with CAI in an amount, per amount of enzyme protein, less than twice the amount of substrate reacting with CAII, through controlling conditions such as substrate concentration and reaction time. Examples of the substrate include p-nitrophenyl esters and α-naphthyl esters, which are different from those described above. Of these, p-nitrophenyl esters are preferred. These esters are preferably derived from a fatty acid, more preferably a C1 to C6 fatty acid. Specifically, p-nitrophenyl acetate is preferred.
The "inhibitor inhibiting a hydrolase other than CA" refers to an inhibitor which can reduce the ratio of amount of the reacted substrate per amount of hydrolase (other than CA) enzyme protein amount to amount of the substrate reacted with CA, through controlling conditions such as substrate concentration, inhibitor concentration and reaction time, as compared with the case in which the relevant inhibitor is not used. Examples of the inhibitor include protease inhibitor cocktail (cat. nos. P2714, P8340, etc., product of Sigma), 4-(2-aminoethyl)benzenesulfonyl fluoride (AEBSF), α-phenylmethanesulfonyl fluoride (PMSF), and pepstatin.

The "drug for enhancing inhibitory activity of the inhibitor" refers to a drug which per se exhibits no inhibitory effect but which enhances inhibitory effect of the hydrolase inhibitor. Examples of the enhancer include aldehydes such as formaldehyde, acetaldehyde, and glutaraldehyde.

The "CA inhibitor inhibiting CAI more potently than CAII" refers to a CA inhibitor exhibiting an inhibitory effect which can be confirmed by that the amount (per enzyme protein) of substrate whose reaction with CAI has been inhibited is twice or more the amount of substrate whose reaction with CAII has been inhibited, through controlling conditions such as substrate concentration, inhibitor concentration and reaction time. Examples of the inhibitor include imidazoles and anions (e.g., $CNS^-$, $CNO^-$, $CN^-$, and $I^-$).

The "CA inhibitor inhibiting both CAI and CAII" refers to a CA inhibitor exhibiting an inhibitory effect which can be confirmed by that the amount (per enzyme protein) of substrate whose reaction with CAI has been inhibited less than twice the amount of substrate whose reaction with CAII has been inhibited, through controlling conditions such as substrate concentration, inhibitor concentration and reaction time, as compared with the case in which the relevant inhibitor is not used. Examples of the inhibitor include amides (e.g., dorzolamide, brinzolamide, acetazolamide, and metazolamide) and carbamoyl phosphate. Of these, acetazolamide is particularly preferred.

Preferred combinations of the substrate, the inhibitor, and the enhancer as shown above (A) to (E) are as follows:

(A) Substrate: o-nitrophenyl ester, particularly o-nitrophenyl acetate as a substrate having higher reactivity with CAI than with CAII;

(B) Substrate: o-nitrophenyl ester, particularly o-nitrophenyl acetate as a substrate having higher reactivity with CAI than with CAII,

Inhibitor: protease inhibitor cocktail, AEBSF, or pepstatin, particularly AEBSF as an inhibitor inhibiting a hydrolase other than CA, and

Aldehyde, particularly formaldehyde as an optional drug for enhancing inhibitory activity of the inhibitor;

(C) Substrate: o-nitrophenyl ester, particularly o-nitrophenyl acetate as a substrate having higher reactivity with CAI than with CAII; and

Inhibitor: amide, particularly acetazolamide as a CA inhibitor inhibiting both CAI and CAII;

(D) Substrate: p-nitrophenyl ester, particularly p-nitrophenyl acetate as a substrate having reactivity with both CAI and CAII, and

Inhibitor: anion, particularly iodide ion as a CA inhibitor inhibiting CAI more potently than CAII; and

(E) Substrate: o-nitrophenyl ester, particularly o-nitrophenyl acetate as a substrate having higher reactivity with CAI than with CAII, and

Inhibitor: anion, particularly iodide ion as a CA inhibitor inhibiting CAI more potently than CAII.

The amount of reacted substrate per amount of enzyme protein may be determined through a conventional method; i.e., determination of rate of enzymatic reaction under predetermined conditions including substrate concentration, enzyme concentration, inhibitor concentration, reaction temperature, reaction pH, and reaction time. In addition to the aforementioned substrates, inhibitors, and enhancers, in the present invention, a substrate decomposed by a hydrolase, an inhibitor inhibiting a hydrolase other than CA, an enhancer enhancing the hydrolase activity inhibitory effect of the inhibitor, or a CA inhibitor not inhibiting a hydrolase other than CA may also be employed as the substrate, inhibitor, or enhancer, so long as these components have the aforementioned characteristics.

No particular limitation is imposed on the method of assaying hydrolase activity of a sample, and any conventional method may be employed depending on the substrate. Specifically, a method employing a spectrophotometer, a method including quantitation of diazonium salt and azo dye which are formed, the pH-stat method, etc. may be employed (see, for example, "Essence of Clinical Test," edited by Masamitsu KANAI (Kanahara & Co., Ltd.), 1996, Dec. 10, 30th edition, p. 312 to 315, and Biochemistry Data Book II, edited by Chemical Society of Japan, p. 6 to 269).

The present invention also provides a kit for determining CAI hydrolase activity comprising any of the aforementioned combinations of (A) to (E). The kit may further contain the below-described reagents. A sample diluent or a substrate liquid to which an inhibitor or an enhancer has been added may also be employed.

(1) Sample diluent
(2) Substrate liquid
(3) Inhibitor liquid (in accordance with need)

In addition to the aforementioned inhibitors, an enhancer may be added to the kit. In accordance with needs, an additional reagent may be appropriately selected-, in accordance with the assay method, from among a diazonium salt reagent, a reaction terminator, a standard enzyme reagent, a sample pretreatment agent, and other reagents, and may be incorporated into the kit of the present invention.

Examples

The present invention will next be described in detail by way of examples, which should not be construed as limiting the invention thereto.

Example 1

<Reagents>

o-Nitrophenyl acetate was employed as a substrate having higher reactivity with CAI than with CAII, and no inhibitor was used.

<Procedure>

(1) By use of purified water, a 25-μg/mL human CAI solution and a 5-μg/mL human CAII solution were prepared.
(2) A 30mM dimethyl malonate buffer (pH: 8) was provided.
(3) By use of purified water, a 9mM o-nitrophenyl acetate solution was prepared. Before dissolution in water, the substrate had been dissolved in advance in acetone. Thus, the solution had an acetone content of 2% (v/v).
(4) Each enzyme was reacted with the substrate. Specifically, solutions of (1), (2), and (3) (each 50 μL) were mixed, and the mixture was allowed to react at room temperature for five minutes. Absorbance of the mixture was measured at 405 nm before and after reaction, and change in absorbance (ΔA) was calculated.
In order to prepare a sample reference, only purified water was used as solution (1). The reference was allowed to react in the same manner as above, and absorbance of the mixture was measured at 405 nm before and after reaction, whereby change in absorbance of the reference was determined. The change in absorbance intrinsic to CA was calculated by the following equation:

<Calculation>

$$\text{Change in CA-intrinsic absorbance} = \Delta A1 - \Delta A3;$$

wherein

ΔA1 represents change in absorbance of a sample consisting of solutions (1), (2), and (3), and
ΔA3 represents change in absorbance of a sample consisting of reference solution (1) and solutions (2) and (3).

<Results>

Table 1 shows the results. As is clear from Table 1, CAI can be virtually determined when o-nitrophenyl acetate-having higher reactivity with CAI than with CAII-was employed as a sole substrate.
Table 1

| Substrate | o-Nitrophenyl acetate | |
|---|---|---|
| Enzyme | CAI | CAII |
| Absorbance change | 0.511 | 0.011 |

Example 2

<Sample>

A human erythrocyte lysate was employed as a sample.

<Reagents>

o-Nitrophenyl acetate was employed as a substrate, and no inhibitor was used.

<Procedure>

(1) A human erythrocyte lysate was diluted 250-fold with purified water, to thereby prepare a sample solution.
(2) A 30mM dimethyl malonate buffer (pH: 8) was provided.
(3) By use of purified water, a 9mM o-nitrophenyl acetate solution was prepared. Before dissolution in water, the substrate had been dissolved in advance in acetone. Thus, the solution had an acetone content of 2% (v/v).
(4) Solutions of (1), (2), and (3) (each 50 $\mu$L) were mixed, and the mixture was allowed to react at room temperature for five minutes. Absorbance of the mixture was measured at 405 nm before and after reaction, and change in absorbance ($\Delta$A) was calculated.

In order to prepare a sample reference, only purified water was used as solution (1). The reference was allowed to react in the same manner as above, and absorbance of the mixture was measured at 405 nm before and after reaction, whereby change in absorbance of the reference was determined. The change in absorbance intrinsic to CAI was calculated by the following equation:

<Calculation>

$$\text{Change in CAI-intrinsic absorbance} = \Delta A1 - \Delta A3;$$

wherein

$\Delta$A1 represents change in absorbance of a sample consisting of solutions (1), (2), and (3), and
$\Delta$A3 represents change in absorbance of a sample consisting of reference solution (1) and solutions (2) and (3).

<Results>

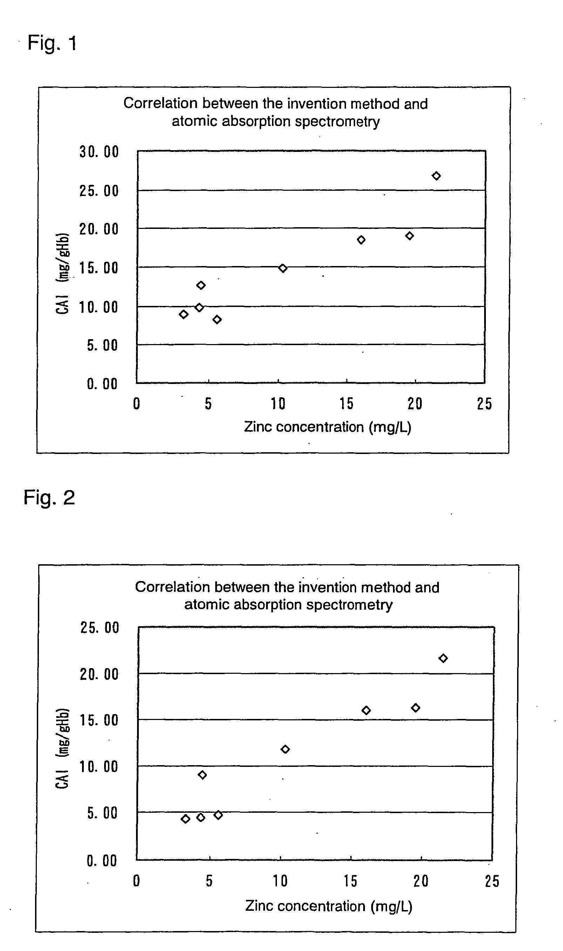

Fig. 1 shows the results. In the graph given in Fig. 1, the Y-axis represents CAI concentration (mg/gHb) of a human erythrocyte lysate. The concentration has been derived from the amount (mg) of CAI contained in the human erythrocyte lysate calculated by use of a 20-$\mu$g/mL human CAI solution as a calibrator, by dividing the amount of CAI by the amount of hemoglobin (Hb). The X-axis represents zinc concentration (mg/L) of a sample determined through atomic absorption spectrometry. The reason for selecting the zinc concentration is that CAI is a zinc enzyme to which over 80% of zinc atoms present in erythrocytes are bound.
As is clear from Fig. 1, a certain level of activity attributed to hydrolases other than CA, corresponding to the $\gamma$-intercept, was found. However, when a substrate having higher reactivity with CAI than with CAII was employed, a linear relationship between CAI concentration and zinc concentration (correlation factor: 0.9380, y-intercept: 6.2524) was established without using an inhibitor. Therefore, the method of the present invention was confirmed to be a practical, CAI-specific assay method which can be applied to specimens containing large amounts of hydrolases, other than CA, such as clinical specimens, and to other conditions where a significant amount of an added substrate is decomposed by such enzymes.

Example 3

<Reagents>

o-Nitrophenyl acetate was employed as a substrate having higher reactivity with CAI than with CAII. In addition, 4-(2-aminoethyl)benzenesulfony fluoride (AEBSF) serving as an inhibitor inhibiting a hydrolase other than CA, and formaldehyde serving as an enhancer of the inhibitor were employed.

**<Procedure>**

(1) By use of purified water, a 25-$\mu$g/mL human CAI solution and a 5-$\mu$g/mL human CAII solution were prepared.
(2) A 30mM dimethyl malonate buffer (pH: 8) including 0.1mM AEBSF, and 50mM formaldehyde were provided.
(3) By use of purified water, a 9mM o-nitrophenyl acetate solution was prepared. Before dissolution in water, the substrate had been dissolved in advance in acetone. Thus, the solution had an acetone content of 2% (v/v).
(4) Each enzyme was reacted with the substrate. Specifically, solutions of (1), (2), and (3) (each 50 $\mu$L) were mixed, and the mixture was allowed to react at room temperature for five minutes. Absorbance of the mixture was measured at 405 nm before and after reaction, and change in absorbance ($\Delta$A) was calculated.

In order to prepare a sample reference, only purified water was used as solution (1). The reference was allowed to react in the same manner as above, and absorbance of the mixture was measured at 405 nm before and after reaction, whereby change in absorbance of the reference was determined. The change in absorbance intrinsic to CA was calculated by the following equation:

<Calculation>

$$\texttt{Change in CA-intrinsic absorbance} = \Delta A1 - \Delta A3;$$

wherein

$\Delta$A1 represents change in absorbance of a sample consisting of solution (1), solution (2), and solution (3), and

$\Delta$A3 represents change in absorbance of a sample consisting of reference solution (1), solution (2), and solution (3).

<Results>

Table 2 shows the results. As is clear from Table 2, CAI can be determined with higher specificity through employment of o-nitrophenyl acetate serving as a substrate having higher reactivity with CAI than with CAII, an inhibitor inhibiting a hydrolase other than CA, and an enhancer of the inhibitor.

Table 2

| Substrate | o-Nitrophenyl acetate |
|---|---|
| Enzyme | CAI CAII |
| Absorbance change | 0.329 0.008 |

Example 4

<Sample>

A human erythrocyte lysate was employed as a sample.

<Reagents>

o-Nitrophenyl acetate was employed as a substrate having higher reactivity with CAI than with CAII. In addition, 4-(2-aminoethyl)benzenesulfony fluoride (AEBSF) serving as an inhibitor inhibiting a hydrolase other than CA, and formaldehyde serving as an enhancer of the inhibitor were employed.

<Procedure>

(1) A human erythrocyte lysate was diluted 250-fold with purified water, to thereby prepare a sample solution.
(2) A 30mM dimethyl malonate buffer (pH: 8) including 0.1mM AEBSF, and 50mM formaldehyde were provided.
(3) By use of purified water, a 9mM o-nitrophenyl acetate solution was prepared. Before dissolution in water, the substrate had been dissolved in advance in acetone. Thus, the solution had an acetone content of 2% (v/v).
(4) Solutions of (1), (2), and (3) (each 50 μL) were mixed, and the mixture was allowed to react at room temperature for five minutes. Absorbance of the mixture was measured at 405 nm before and after reaction, and change in absorbance (ΔA) was calculated.

In order to prepare a sample reference, only purified water was used as solution (1). The reference was allowed to react in the same manner as employed above, and absorbance of the mixture was measured at 405 nm before and after reaction, whereby change in absorbance of the reference was determined. The change in absorbance intrinsic to CAI was calculated by the following equation:

<Calculation>

$$\text{Change in CAI-intrinsic absorbance} = \Delta A1 - \Delta A3;$$

: wherein

ΔA1 represents change in absorbance of a sample consisting of solutions (1), (2), and (3), and
ΔA3 represents change in absorbance of a sample consisting of reference solution (1) and solutions (2) and (3) .

<Results>

Fig. 2 shows the results. In the graph given in Fig. 2, the Y-axis represents CAI concentration (mg/gHb) of a human erythrocyte lysate. The concentration has been derived from the amount (mg) of CAI contained in the human erythrocyte lysate calculated by use of a 20-μg/mL human CAI solution as a calibrator, by dividing the amount of CAI by the amount of hemoglobin (Hb). The X-axis represents zinc concentration (mg/L) of a sample determined through atomic absorption spectrometry. The reason for selecting the zinc concentration is that CAI is a zinc enzyme to which over 80% of zinc atoms present in erythrocytes are bound.
As is clear from Fig. 2, when an inhibitor inhibiting a hydrolase other than CA and an enhancer of the inhibitor were employed, noise hydrolase activity was suppressed, and a linear relationship between CAI concentration and zinc concentration (correlation factor: 0.9577, γ-intercept: 2.0898) was established. Therefore, the method of the present invention was confirmed to be a practical, CAI-specific assay method which can be applied to specimens containing large amounts of hydrolases, other than CA, such as clinical specimens, and other conditions where a significant amount of an added substrate is decomposed by such enzymes.

Example 5

<Reagents>

o-Nitrophenyl acetate was employed as a substrate having higher reactivity with CAI than with CAII. As an inhibitor, acetazolamide, which inhibits both CAI and CAII, was used in combination. A reference substrate was produced from p-nitrophenyl acetate-a substrate having low specificity to CAI (reactive with both CAI and CAII)-and the above acetazolamide serving as a CA inhibitor in combination.

**<Procedure>**

(1) By use of purified water, a 25-$\mu$g/mL human CAI solution and a 5-$\mu$g/mL human CAII solution were prepared.

(2) A 3mM acetazolamide solution was prepared by use of a 30mM dimethyl malonate buffer (pH 8). Before dissolution in the buffer, acetazolamide had been dissolved in advance in dimethyl sulfoxide (DMSO). Thus, the solution has a DMSO content of 0.15% (v/v).

(3) By use of purified water, a 6mM o-nitrophenyl acetate solution and a 3mM p-nitrophenyl acetate solution were prepared. Before dissolution in water, each substrate had been dissolved in advance in acetone. Thus, each solution had an acetone content of 2% (v/v).

(4) Each enzyme was reacted with each substrate. Specifically, solutions of (1), (2), and (3) (each 50 $\mu$L) were mixed, and the mixture was allowed to react at room temperature for five minutes. Absorbance of the mixture was measured at 405 nm before and after reaction, and change in absorbance ($\Delta$A) was calculated.

In order to prepare a sample reference and an inhibitor reference, only purified water and 0.15% (v/v) DMSO-containing 30mM dimethyl malonate buffer were used as reference solutions (1) and (2), respectively. Each of the references was allowed to react in the same manner as above, and absorbance of the mixture was measured, whereby change in absorbance of the reference was determined. The change in absorbance intrinsic to an enzyme specifically inhibited by acetazolamide (i.e., intrinsic to CA) was calculated by the following equation:

**<Calculation>**

Change in CA-intrinsic absorbance = [$\Delta$A1-$\Delta$A3]-[$\Delta$A2-$\Delta$A4];

wherein

$\Delta$A1 represents change in absorbance of a sample consisting of solution (1), reference solution (2), and solution (3);

$\Delta$A2 represents change in absorbance of a sample consisting of solutions (1), (2), and (3);

$\Delta$A3 represents change in absorbance of a sample consisting of reference solutions (1) and (2), and solution (3); and

$\Delta$A4 represents change in absorbance of a sample consisting of reference solution (1) and solutions (2) and (3).

<Results>

Table 3 shows the results. As is clear from Table 3, in the case where p-nitrophenyl acetate and acetazolamide were used in combination, reactivity of p-nitrophenyl acetate serving as a substrate with CAI was lower than twice the reactivity with CAII. Thus, p-nitrophenyl acetate failed to have CAI-specific reactivity, and was reacted with both CAI and CAII. Therefore, CAI cannot be specifically determined. In contrast, in the case where o-nitrophenyl acetate and acetazolamide were used in combination, reactivity of o-nitrophenyl acetate serving as a substrate with CAI was higher than twice the reactivity with CAII. Therefore, CAI can be specifically determined.

Table 3

| Substrate | o-Nitrophenyl acetate | p-Nitrophenyl acetate |
|---|---|---|
| Enzyme | CAI    CAII | CAI    CAII |
| Absorbance change | 0.285 0.004 | 0.283 0.162 |

**Example 6**

<Sample>

A human erythrocyte lysate was employed as a sample.

<Reagents>

o-Nitrophenyl acetate was used as a substrate, and acetazolamide was used as an inhibitor, in combination.

<Procedure>

(1) A human erythrocyte lysate was diluted 250-fold with purified water, to thereby prepare a sample solution.
(2) A 3mM acetazolamide solution was prepared by use of a 30mM dimethyl malonate buffer (pH 8). Before dissolution in the buffer, acetazolamide had been dissolved in advance in dimethyl sulfoxide (DMSO). Thus, the solution has a DMSO content of 0.15% (v/v).
(3) By use of purified water, a 9mM o-nitrophenyl acetate solution was prepared. Before dissolution in water, the substrate had been dissolved in advance in acetone. Thus, the solution had an acetone content of 2% (v/v).
(4) Solutions of (1), (2), and (3) (each 50 $\mu$L) were mixed, and the mixture was allowed to react at room temperature for five minutes. Absorbance of the mixture was measured at 405 nm before and after reaction, and change in absorbance (AA) was calculated.

In order to prepare a sample reference and an inhibitor reference, only purified water and 0.15% (v/v) DMSO-containing 30mM dimethyl malonate buffer were used as reference solutions (1) and (2), respectively. Each of the references was allowed to react in the same manner as above, and absorbance of the mixture was measured, whereby change in absorbance of the reference was determined. The change in absorbance intrinsic to an enzyme specifically inhibited by acetazolamide (i.e., intrinsic to CAI) was calculated by the following equation:

<Calculation>

$$\texttt{Change in CA-intrinsic absorbance = } [\Delta A1 - \Delta A3] - [\Delta A2 - \Delta A4];$$

wherein

$\Delta A1$ represents change in absorbance of a sample consisting of solution (1), reference solution (2), and solution (3);
$\Delta A2$ represents change in absorbance of a sample consisting of solutions (1), (2), and (3);
$\Delta A3$ represents change in absorbance of a sample consisting of reference solutions (1) and (2), and solution (3); and
$\Delta A4$ represents change in absorbance of a sample consisting of reference solution (1) and solutions (2) and (3).

<Results>

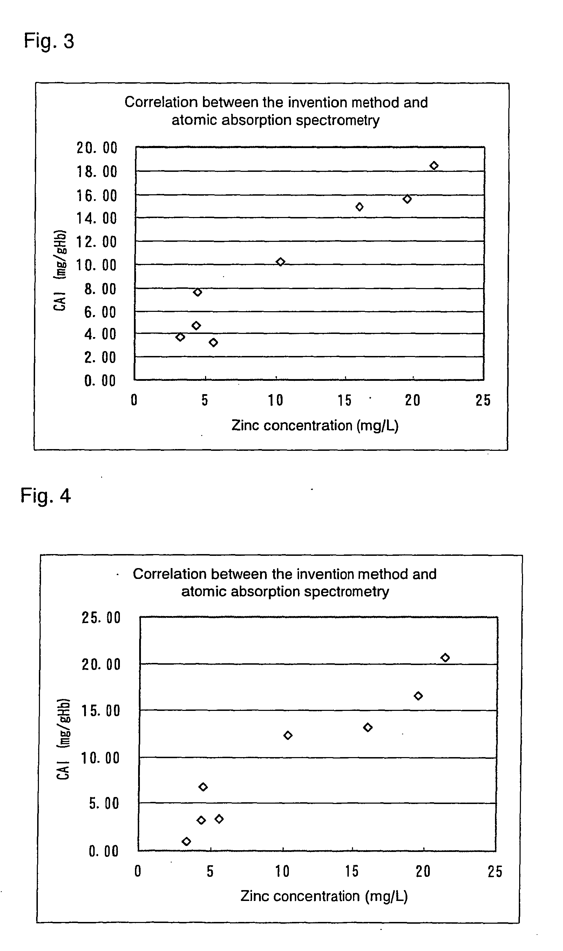

Fig. 3 shows the results. In the graph given in Fig. 3, Y-axis represents CAI concentration (mg/gHb) of a human erythrocyte lysate. The concentration has been derived from the amount (mg) of CAI contained in the human erythrocyte lysate calculated by use of a 20-$\mu$g/mL human CAI solution as a calibrator, by dividing the amount of CAI by the amount of hemoglobin (Hb). X-axis represents zinc concentration (mg/L) of a sample determined through atomic absorption spectrometry. The reason for selecting the zinc concentration is that CAI is a zinc enzyme to which over 80% of zinc atoms present in erythrocytes are bound.
As is clear from Fig. 3, when a substrate having higher reactivity with CAI than with CAII and a CA inhibitor inhibiting both CAI and CAII were employed in combination, CA-specific assay was realized similar to Example 4. A linear relationship between CAI concentration and zinc concentration (correlation factor: 0.9642, y-intercept: 1.6282) was established. Therefore, the method of the present invention was confirmed to be a practical, CAI-specific assay method which can be applied to specimens containing large amounts of hydrolases, other than CA, such as clinical specimens, and other conditions where a significant amount of an added substrate is decomposed by such enzymes.

Example 7

<Reagents>

p-Nitrophenyl acetate serving as a substrate reacting with both CAI and CAII and an iodide (I⁻) serving as a CA inhibitor inhibiting CAI more potently than CAII were employed in combination.

<Procedure>

(1) By use of purified water, a 25-μg/mL human CAI solution and a 5-μg/mL human CAII solution were prepared.
(2) A 3mM potassium iodide solution was prepared by use of a 30mM dimethyl malonate buffer (pH 8).
(3) By use of purified water, a 3mM p-nitrophenyl acetate solution was prepared. Before dissolution in water, the substrate had been dissolved in advance in acetone. Thus, the solution had an acetone of 2% (v/v).
(4) Each enzyme was reacted with each substrate. Specifically, solutions of (1), (2), and (3) (each 50 μL) were mixed, and the mixture was allowed to react at room temperature for five minutes. Absorbance of the mixture was measured at 405 nm before and after reaction, and change in absorbance (ΔA) was calculated.

In order to prepare a sample reference and an inhibitor reference, only purified water and 30mM dimethyl malonate buffer were used as reference solutions (1) and (2), respectively. Each of the references was allowed to react in the same manner as employed above, and absorbance of the mixture was measured, whereby change in absorbance of the reference was determined. The change in absorbance intrinsic to an enzyme specifically inhibited by iodide (i.e., intrinsic to CA) was calculated by the following equation:

<Calculation>

$$\text{Change in CA-intrinsic absorbance} = [\Delta A1 - \Delta A3] - [\Delta A2 - \Delta A4];$$

wherein

ΔA1 represents change in absorbance of a sample consisting of solution (1), reference solution (2), and solution (3);
ΔA2 represents change in absorbance of a sample consisting of solutions (1), (2), and (3);
ΔA3 represents change in absorbance of a sample consisting of reference solutions (1) and (2), and solution (3); and
ΔA4 represents change in absorbance of a sample consisting of reference solution (1) and solutions (2) and (3).

<Results>

Table 4 shows the results. As is clear from Table 4, even when p-nitrophenyl acetate serving as a substrate reacting with both CAI and CAII is employed, if an iodide (I ) serving as a CA inhibitor inhibiting CAI more potently than CAII is employed in combination, CAI can be specifically determined.

Table 4

| Substrate | p-Nitrophenyl acetate | |
|---|---|---|
| Enzyme | CAI | CAII |
| Absorbance change | 0.052 | 0.005 |

Example 8

<Sample>

A human erythrocyte lysate was employed as a sample.

<Reagents>

p-Nitrophenyl acetate was used as a substrate, and an iodide ion was used as a CA inhibitor in combination.

<Procedure>

(1) A human erythrocyte lysate was diluted 250-fold with purified water, to thereby prepare a sample solution.
(2) A 3mM potassium iodide solution was prepared by use of a 30mM dimethyl malonate buffer (pH 8).
(3) By use of purified water, a 3mM p-nitrophenyl acetate solution was prepared. Before dissolution in water, the substrate had been dissolved in advance in acetone. Thus, the solution had an acetone of 2% (v/v).
(4) Solutions of (1), (2), and (3) (each 50 $\mu$L) were mixed, and the mixture was allowed to react at room temperature for five minutes. Absorbance of the mixture was measured at 405 nm before and after reaction, and change in absorbance ($\Delta$A) was calculated.

In order to prepare a sample reference and an inhibitor reference, only purified water and 30mM dimethyl malonate buffer were used as reference solutions (1) and (2), respectively. Each of the references was allowed to react in the same manner as employed above, and absorbance of the mixture was measured, whereby change in absorbance of the reference was determined. The change in absorbance intrinsic to an enzyme specifically inhibited by iodide (i.e., intrinsic to CAI) was calculated by the following equation:

<Calculation>

$$\texttt{Change in CAI-intrinsic absorbance = [}\Delta\texttt{A1-}\Delta\texttt{A3]-[}\Delta\texttt{A2-}\Delta\texttt{A4];}$$

wherein

$\Delta$A1 represents change in absorbance of a sample consisting of solution (1), reference solution (2), and solution (3);
$\Delta$A2 represents change in absorbance of a sample consisting of solutions (1), (2), and (3) ;
$\Delta$A3 represents change in absorbance of a sample consisting of reference solutions (1) and (2), and solution (3); and
$\Delta$A4 represents change in absorbance of a sample consisting of reference solution (1) and solutions (2) and (3).

<Results>

Fig. 4 shows the results. In the graph given in Fig. 4, Y-axis represents CAI concentration (mg/gHb) of a human erythrocyte lysate. The concentration has been derived from the amount (mg) of CAI contained in the human erythrocyte lysate calculated by use of a 20-$\mu$g/mL human CAI solution as a calibrator, by dividing the amount of CAI by the amount of hemoglobin (Hb). X-axis represents zinc concentration (mg/L) of a sample determined through atomic absorption spectrometry. The reason for selecting the zinc concentration is that CAI is a zinc enzyme to which over 80% of zinc atoms present in erythrocytes are bound.

As is clear from Fig. 4, even when a substrate reacting with both CAI and CAII is employed, if a CA inhibitor inhibiting CAI more potently than CAII is employed, the same determination as performed in Example 6 can be realized. In addition, a linear relationship between CAI concentration and zinc concentration (correlation factor: 0.9588, $\gamma$-intercept: -0.1615) was established. Therefore, the method of the present invention was confirmed to be a practical, CAI-specific assay method which can be applied to specimens containing large amounts of hydrolases, other than CA, such as clinical specimens, and other conditions where a significant amount of an added substrate is decomposed by such enzymes.

Example 9

<Reagents>

o-Nitrophenyl acetate was used as a substrate having higher reactivity with CAI than with CAII, and in combination, an iodide ion (I⁻) was used as a CA inhibitor inhibiting CAI more potently than CAII.

<Procedure>

(1) By use of purified water, a 25-μg/mL human CAI solution and a 5-μg/mL human CAII solution were prepared.
(2) A 300mM potassium iodide solution was prepared by use of a 30mM dimethyl malonate buffer (pH 8).
(3) By use of purified water, a 9mM o-nitrophenyl acetate solution was prepared. Before dissolution in water, the substrate had been dissolved in advance in acetone. Thus, the solution had an acetone content of 2% (v/v).
(4) Solutions of (1), (2), and (3) (each 50 μL) were mixed, and the mixture was allowed to react at room temperature for five minutes. Absorbance of the mixture was measured at 405 nm before and after reaction, and change in absorbance (ΔA) was calculated.

In order to prepare a sample reference and an inhibitor reference, only purified water and 30mM dimethyl malonate buffer were used as reference solutions (1) and (2), respectively. Each of the references was allowed to react in the same manner as employed above, and absorbance of the mixture was measured, whereby change in absorbance of the reference was determined. The change in absorbance intrinsic to an enzyme specifically inhibited by iodide (i.e., intrinsic to CA) was calculated by the following equation:

<Calculation>

$$\text{Change in CA-intrinsic absorbance} = [\Delta A1 - \Delta A3] - [\Delta A2 - \Delta A4];$$

wherein

ΔA1 represents change in absorbance of a sample consisting of solution (1) , reference solution (2), and solution (3);
ΔA2 represents change in absorbance of a sample consisting of solutions (1) , (2), and (3);
ΔA3 represents change in absorbance of a sample consisting of reference solutions (1) and (2), and solution (3); and
ΔA4 represents change in absorbance of a sample consisting of reference solution (1) and solutions (2) and (3).

<Results>

Table 5 shows the results. As is clear from Table 5, when o-nitrophenyl acetate serving as a substrate having higher reactivity with CAI than with CAII, and an iodide (I⁻) serving as a CA inhibitor inhibiting CAI more potently than CAII are employed in combination, CAI can be specifically determined.
Table 5

| Substrate | o-Nitrophenyl acetate | |
|---|---|---|
| Enzyme | CAI | CAII |
| Absorbance change | 0.504 | 0.009 |

**Example 10**

**<Sample>**

A human erythrocyte lysate was employed as a sample. <Reagents>

o-Nitrophenyl acetate serving as a substrate and an iodide ion serving as a CA inhibitor were employed in combination.

<Procedure>

(1) A human erythrocyte lysate was diluted 250-fold with purified water, to thereby prepare a sample solution.
(2) A 300mM potassium iodide solution was prepared by use of a 30mM dimethyl malonate buffer (pH 8).
(3) By use of purified water, a 9mM o-nitrophenyl acetate solution was prepared. Before dissolution in water, the substrate had been dissolved in advance in acetone. Thus, the solution had an acetone content of 2% (v/v).
(4) Solutions of (1), (2), and (3) (each 50 $\mu$L) were mixed, and the mixture was allowed to react at room temperature for five minutes. Absorbance of the mixture was measured at 405 nm before and after reaction, and change in absorbance ($\Delta$A) was calculated.

In order to prepare a sample reference and an inhibitor reference, only purified water and 30mM dimethyl malonate buffer were used as reference solutions (1) and (2), respectively. Each of the references was allowed to react in the same manner as employed above, and absorbance of the mixture was measured, whereby change in absorbance of the reference was determined. The change in absorbance intrinsic to an enzyme specifically inhibited by iodide (i.e., intrinsic to CAI) was calculated by the following equation:

<Calculation>

$$\text{Change in CAI-intrinsic absorbance} = [\Delta A1 - \Delta A3] - [\Delta A2 - \Delta A4];$$

wherein

$\Delta$A1 represents change in absorbance of a sample consisting of solution (1), reference solution (2), and solution (3);
$\Delta$A2 represents change in absorbance of a sample consisting of solutions (1), (2), and (3);
$\Delta$A3 represents change in absorbance of a sample consisting of reference solutions (1) and (2), and solution (3); and
$\Delta$A4 represents change in absorbance of a sample consisting of reference solution (1) and solutions (2) and (3).

<Results>

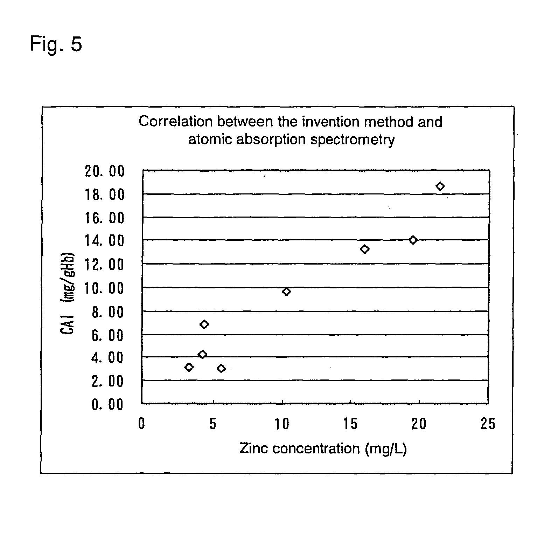

Fig. 5 shows the results. In the graph given in Fig. 5, Y-axis represents CAI concentration (mg/gHb) of a human erythrocyte lysate. The concentration has been derived from the amount (mg) of CAI contained in the human erythrocyte lysate calculated by use of a 20-$\mu$g/mL human CAI solution as a calibrator, by dividing the amount of CAI by the amount of hemoglobin (Hb). X-axis represents zinc concentration (mg/L) of a sample determined through atomic absorption spectrometry. The reason for selecting the zinc concentration is that CAI is a zinc enzyme to which over 80% of zinc atoms present in erythrocytes are bound.
As is clear from Fig. 5, when a substrate having higher reactivity with CAI than with CAII and a CA inhibitor inhibiting CAI more potently than CAII were employed, a linear relationship between CAI concentration and zinc concentration (correlation factor: 0.9618, y-intercept: 1.1661) was established. Therefore, the method of the present invention was confirmed to be a practical, CAI-specific assay method which can be applied to specimens containing large amounts of hydrolases, other than CA, such as clinical specimens, and other conditions where a significant amount of an added substrate is decomposed by such enzymes.

Brief Description of the Drawings

[Fig. 1] Fig. 1 is a graph showing the determination results obtained in Example 2. The X-axis represents zinc concentration determined through atomic absorption spectrometry (reference), and the Y-axis represents CAI concentration determined through the method of the invention. Measurement results of eight specimens are plotted.
[Fig. 2] Fig. 2 is a graph showing the determination results obtained in Example 4. The X-axis represents zinc concentration determined through atomic absorption spectrometry (reference), and the Y-axis represents CAI concentration determined through the method of the invention. Measurement results of eight specimens are plotted.

[Fig. 3] Fig. 3 is a graph showing the determination results obtained in Example 6. The X-axis represents zinc concentration determined through atomic absorption spectrometry (reference), and the Y-axis represents CAI concentration determined through the method of the invention. Measurement results of eight specimens are plotted.

[Fig. 4] Fig. 4 is a graph showing the determination results obtained in Example 8. The X-axis represents zinc concentration determined through atomic absorption spectrometry (reference), and the Y-axis represents CAI concentration determined through the method of the invention. Measurement results of eight specimens are plotted.

[Fig. 5] Fig. 5 is a graph showing the determination results obtained in Example 10. The X-axis represents zinc concentration determined through atomic absorption spectrometry (reference), and the Y-axis represents CAI concentration determined through the method of the invention. Measurement results of eight specimens are plotted.

**Claims**

1. A method for determining hydrolase activity of carbonic anhydrase I (CAI) in a sample, **characterized in that** the method employs, as a substrate or a combination of a substrate and an inhibitor, any of the following (A) to (E):

   (A) Substrate: a substrate having higher reactivity with CAI than with CAII;
   (B) Substrate: a substrate having higher reactivity with CAI than with CAII, and
   Inhibitor: an inhibitor inhibiting a hydrolase other than CA;
   (C) Substrate: a substrate having higher reactivity with CAI than with CAII, and
   Inhibitor: a CA inhibitor inhibiting both CAI and CAII;
   (D) Substrate: a substrate having reactivity with both CAI and CAII, and
   Inhibitor: a CA inhibitor inhibiting CAI more potently than CAII; and
   (E) Substrate: a substrate having higher reactivity with CAI than with CAII, and
   Inhibitor: a CA inhibitor inhibiting CAI more potently than CAII.

2. A method as described in claim 1, wherein the substrate (A) or the combination of the substrate and the inhibitor (B) is employed, and hydrolase activity of the sample is employed as CAI hydrolase activity.

3. A method as described in claim 1 or 2, wherein the combination of the substrate and the inhibitor (B) and a drug for enhancing inhibitory activity of the inhibitor are employed in combination.

4. A method as described in claim 1, wherein hydrolase activity of the sample is determined by use of the combination of the substrate and the inhibitor (C), (D), or (E) in the presence and in the absence of the inhibitor, and the difference between the two activity values is employed as CAI hydrolase activity.

5. A method as described in any one of claims 1 to 4, wherein hydrolase activity is esterase activity.

6. A method as described in any one of claims 1 to 4, wherein the substrate having higher reactivity with CAI than with CAII is an o-nitrophenyl ester.

7. A method as described in claim 1 or 4, wherein the substrate having reactivity with both CAI and CAII is a p-nitrophenyl ester.

8. A method as described in claim 1 or 4, wherein the CA inhibitor inhibiting both CAI and CAII is an amide.

9. A method as described in claim 1 or 4, wherein the CA inhibitor inhibiting CAI more potently than CAII is an anion.

10. A method as described in any one of claims 1 to 3, wherein the inhibitor inhibiting a hydrolase other than CA is at lease one species selected from among protease inhibitor cocktail, 4-(2-aminoethyl)benzenesulfonyl fluoride (AEBSF), $\alpha$-phenylmethanesulfonyl fluoride (PMSF), and pepstatin.

11. A method as described in claim 3, wherein the drug for enhancing inhibitory activity of the inhibitor is an aldehyde.

12. A kit for determining hydrolase activity of CAI in a sample, **characterized in that** the kit comprises, as a substrate or a combination of a substrate and an inhibitor, any of the aforementioned (A) to (E):

   (A) Substrate: a substrate having higher reactivity with CAI than with CAII;

(B) Substrate: a substrate having higher reactivity with CAI than with CAII, and
Inhibitor: an inhibitor inhibiting a hydrolase other than CA;
(C) Substrate: a substrate having higher reactivity with CAI than with CAII, and
Inhibitor: a CA inhibitor inhibiting both CAI and CAII;
(D) Substrate: a substrate having reactivity with both CAI and CAII, and
Inhibitor: a CA inhibitor inhibiting CAI more potently than CAII; and
(E) Substrate: a substrate having higher reactivity with CAI than with CAII, and
Inhibitor: a CA inhibitor inhibiting CAI more potently than CAII.

13. A kit as described in claim 12, wherein the substrate (A) or the combination of the substrate and the inhibitor (B) is employed, and hydrolase activity of the sample is employed as CAI hydrolase activity.

14. A kit as described in claim 12 or 13, wherein the combination of the substrate and the inhibitor (B) and a drug for enhancing inhibitory activity of the inhibitor are employed in combination.

15. A kit as described in claim 12, wherein hydrolase activity of the sample is determined by use of the combination of the substrate and the inhibitor (C), (D), or (E) in the presence and in the absence of the inhibitor, and the difference between the two activity values is employed as CAI hydrolase activity.

16. A kit as described in any one of claims 12 to 15, wherein hydrolase activity is esterase activity.

17. A kit as described in any one of claims 12 to 15, wherein the substrate having higher reactivity with CAI than with CAII is an o-nitrophenyl ester.

18. A kit as described in claim 12 or 15, wherein the substrate having reactivity with both CAI and CAII is a p-nitrophenyl ester.

19. A kit as described in claim 12 or 15, wherein the CA inhibitor inhibiting both CAI and CAII is an amide.

20. A kit as described in claim 12 or 15, wherein the CA inhibitor inhibiting CAI more potently than CAII is an anion.

21. A kit as described in any one of claims 12 to 14, wherein the inhibitor inhibiting a hydrolase other than CA is at lease one species selected from among protease inhibitor cocktail, 4-(2-aminoethyl)benzenesulfonyl fluoride (AEBSF), $\alpha$-phenylmethanesulfonyl fluoride (PMSF), and pepstatin.

22. A kit as described in claim 14, wherein the drug for enhancing inhibitory activity of the inhibitor is an aldehyde.

Fig. 1

Correlation between the invention method and atomic absorption spectrometry

Fig. 2

Correlation between the invention method and atomic absorption spectrometry

## Fig. 3

Correlation between the invention method and
atomic absorption spectrometry

## Fig. 4

Correlation between the invention method and
atomic absorption spectrometry

Fig. 5

**EP 1 734 132 A1**

## INTERNATIONAL SEARCH REPORT

| | International application No. |
|---|---|
| | PCT/JP2005/006669 |

**A. CLASSIFICATION OF SUBJECT MATTER**
Int.Cl⁷ C12Q1/44//G01N33/49

According to International Patent Classification (IPC) or to both national classification and IPC

**B. FIELDS SEARCHED**

Minimum documentation searched (classification system followed by classification symbols)
Int.Cl⁷ C12Q1/44//G01N33/49

Documentation searched other than minimum documentation to the extent that such documents are included in the fields searched
Jitsuyo Shinan Koho          1922-1996    Jitsuyo Shinan Toroku Koho    1996-2005
Kokai Jitsuyo Shinan Koho    1971-2005    Toroku Jitsuyo Shinan Koho    1994-2005

Electronic data base consulted during the international search (name of data base and, where practicable, search terms used)
WPI(STN), BIOSIS(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| Y | Tohoku J.Exp.Med., Vol.178, (1996), pages 345 to 356 | 1-22 |
| Y | T. KONDO et al., "Estimations of active and inactive carbonic anhydrase isozyme B in human red cells.", Clinica Chimica Acta, Vol.60, (1975), pages 347 to 353 | 1-22 |

☐ Further documents are listed in the continuation of Box C.     ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 April, 2005 (28.04.05) | 24 May, 2005 (24.05.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

20

**EP 1 734 132 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Tohoku J. Exp. Med.,* 1996, vol. 178, 345-356
- *The Journal of Biological Chemistry,* 1967, vol. 242 (18), 4221-4229
- *Clinica Chimica Acta,* 1975, vol. 60, 347-353
- *The Journal of Biological Chemistry,* 1966, vol. 241 (10), 5137-5149
- Essence of Clinical Test. Kanahara & Co., Ltd, 10 December 1996, 312-315
- Biochemistry Data Book II. Chemical Society of Japan, 6-269